# EUROPÄISCHE PATENTSCHRIFT

(11) **EP 0 673 361 B1**
(45) Veröffentlichungstag und Bekanntmachung des Hinweises auf die Patenterteilung: **19.03.1997**
(21) Anmeldenummer: 93923473.8
(22) Anmeldetag: 15.10.1993
(51) Int. Cl.: C07C 231/02

(54) **VERFAHREN ZUR HERSTELLUNG VON N-ALKANOYL-POLYHYDROXYALKYLAMINEN**
PROCESS FOR PREPARING N-ALKANOYL-POLYHYDROXYALKYLAMINES
PROCEDE DE PREPARATION DE N-ALCANOYL POLYHYDROXYALKYLAMINES

(30) Priorität: 23.10.1992 DE 4235783
(43) Veröffentlichungstag der Anmeldung: 27.09.1995
(73) Patentinhaber: BASF Aktiengesellschaft, 67063 Ludwigshafen (DE)
(72) Erfinder: STRECKER, Beate, D-67059 Ludwigshafen (DE); WOLF, Helmut, D-67454 Hassloch (DE); WOLF, Gerhard, D-68775 Ketsch (DE); OFTRING, Alfred, D-67098 Bad Dürkheim (DE); BECHTOLSHEIMER, Hans-Heinrich, D-67596 Dittelsbach-Hessloch (DE); HERTEL, Dieter, D-69181 Leimen (DE)
(86) Internationale Anmeldenummer: EP9302852
(87) Internationale Veröffentlichungsnummer: WO9410130

(56) Entgegenhaltungen:
- EP-A- 0 285 768
- WO-A-92/06073
- US-A- 2 703 798

## Beschreibung

Die vorliegende Erfindung betrifft ein verbessertes Verfahren zur Herstellung von N-Alkanoyl-polyhydroxyalkylaminen durch Umsetzung von Polyhydroxyalkylaminen mit Carbonsäurealkylestern in Gegenwart eines basischen Katalysators.

N-Alkanoyl-polyhydroxyalkylamine, welche hauptsächlich als oberflächenaktive Mittel in Wasch- und Reinigungsmittelformulierungen verwendet werden, sind seit langem bekannt und werden häufig aus den oben genannten Ausgangsstoffen hergestellt.

So lehrt die Schrift US-A 2 703 798 (1) die Umsetzung von N-Monoalkylglucaminen mit aliphatischen Estern von Fettsäuren bei Temperaturen von 140 bis 230°C; dabei werden die Komponenten gemeinsam vorgelegt, bevor die Reaktion durch Erhitzen gestartet wird.

Aus der EP-A 285 768 (2) ist bekannt, daß man die Umsetzung von Fettsäuren bzw. Fettsäureestern mit gegebenenfalls N-substituierten Polyhydroxyalkylaminen in der Schmelze, gegebenenfalls in Gegenwart von alkalischen Katalysatoren, durchführen kann; ein typisches Beispiel ist die Herstellung von N-Methyl-Kokosfettsäureglucamid aus Kokosfettsäuremethylester und N-Methylglucamin unter Zusatz von Natriummethylat durch Erhitzen beider Komponenten auf 135°C.

Die WO 92/06071 (3) betrifft ein Verfahren zur Herstellung oberflächenaktiver linearer Glucamide aus N-Alkylglucaminen und Fettsäureestern unter Zusatz bestimmter Phasentransfer-Katalysatoren; dabei wird zuerst durch Erwärmen und Mischen beider Komponenten ein Zweiphasengemisch hergestellt und dann durch Zugabe des Katalysators die Umsetzung gestartet und bei Temperaturen von 120 bis 200°C durchgeführt.

Aus der WO 92/06073 (4) ist bekannt, Polyhydroxyfettsäureamide aus N-Alkyl-polyhydroxyaminen und Fettsäureestern in einem hydroxylgruppenhaltigen oder alkoxylierten Lösungsmittel wie Methanol, Propylenglykol oder einem ethoxylierten Alkohol in Gegenwart eines basischen Katalysators bei Temperaturen von 40 bis 100°C herzustellen.

Nachteilig an den aus dem Stand der Technik bekannten Verfahren ist, daß das erhaltene Produkt in der Regel einen hohen Anteil an Ausgangsmaterial und Nebenprodukten enthält; dies trifft insbesondere auf die aus (1) bis (3) bekannten Verfahren zu. Bei Verwendung von organischen Lösungsmitteln gemäß (4) müssen diese nach beendeter Reaktion aufwendig durch Destillation aus dem Reaktionsgemisch entfernt werden; dabei kann neben dem Energiemehraufwand für die Abtrennung auch noch die Umweltproblematik bei der Aufarbeitung oder Entsorgung dieser Lösungsmittel hinzukommen. Ein weiterer Nachteil der aus dem Stand der Technik bekannten Verfahren ist häufig auch die Neigung des Reaktionsgemisches, im Reaktor Verklumpungen und Anbackungen zu erzeugen, was die Einhaltung der Reaktionsbedingungen und somit die Reproduzierbarkeit der Produkteigenschaften erschwert und außerdem Reinigungsprobleme des Reaktors mit sich bringt.

Aufgabe der vorliegenden Erfindung war es somit, den geschilderten Mängeln des Standes der Technik abzuhelfen.

Demgemäß wurde ein Verfahren zur Herstellung von N-Alkanoyl-polyhydroxyalkylaminen der allgemeinen Formel I 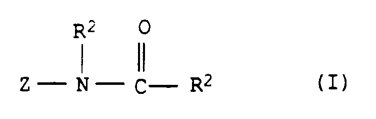 in der
- Z: für den Polyhydroxyalkylrest eines Mono- oder Oligosaccharides steht,
- R¹: Wasserstoff oder C₁-bis C₈-Alkyl bedeutet und
- R²: C₁ bis C₂₁-Alkyl bezeichnet,
durch Umsetzung von Polyhydroxyalkylaminen der allgemeinen Formel II

Z-NH-R¹ (II)

mit Carbonsäurealkylestern der allgemeinen Formel III 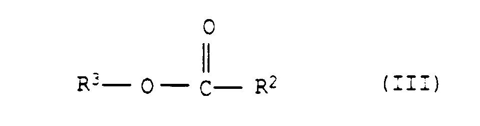 in der R³ einen C₁-bis C₄-Alkylrest bedeutet, in Gegenwart eines basischen Katalysators gefunden, welches dadurch gekennzeichnet ist, daß man
(a) die Gesamtmenge des Esters III vorlegt, auf die Umsetzungstemperatur erwärmt und das Amin II in Form einer Schmelze unter Fortschreiten der Umsetzung zudosiert, wobei gebildeter Alkohol R³-OH fortlaufend abdestilliert wird,
(b) die Umsetzung bei einer Temperatur von 55 bis 110°C durchführt und
(c) die Umsetzung in Abwesenheit von organischen Lösungsmitteln vornimmt, ausgenommen wenn diese organischen Lösungsmittel in der Funktion als Dispergiermittel verwendet werden.

Das erfindungsgemäße Verfahren wird gemäß Maßnahme (a) dergestalt durchgeführt, daß die Gesamtmenge des Esters III im Reaktor vorgelegt und auf die zur Reaktion notwendige Temperatur erwärmt wird. Danach wird das Amin II portionsweise oder vorzugsweise kontinuierlich so zudosiert, daß die Temperatur im Reaktor konstant bleibt oder sich zumindest in den angegebenen Intervallen gemäß (b) bewegt.

Das Amin II wird als Schmelze, deren Temperatur über der Temperatur des Reaktionsgemisches im Reaktor liegen kann, zudosiert. Das verwendete Amin II braucht hierfür nicht speziell gereinigt zu werden, man kann die bei der reduktiven Aminierung von Zuckerderivaten anfallenden technischen Gemische einsetzen. Nur müssen diese Ausgangsmaterialien ausreichend wasserfrei sein, der Wassergehalt sollte < 1 Gew.-%, insbesondere < 0,5 Gew.-% betragen. Hierzu kann man technische Materialien nach den üblichen Methoden entwässern.

Es ist prinzipiell auch möglich, das Amin II als Feststoff über eine Feststoffdosiervorrichtung zuzugeben, nur erfordert dies einen vergleichsweise höheren technischen Aufwand und ist somit unwirtschaftlicher.

Während der Umsetzung gebildeter Alkohol R³-OH und eventuell mit dem basischen Katalysator oder anderen Hilfsstoffen eingebrachte kleine Mengen an Lösungsmitteln werden fortlaufend abdestilliert. Um dieses Abdestillieren zu erleichtern, empfiehlt es sich, die Umsetzung bei vermindertem Druck vorzunehmen, etwa bei 20 bis 500 mbar, vorzugsweise bei 30 bis 300 mbar, insbesondere bei 50 bis 150 mbar.

Gemäß Maßnahme (b) wird die Umsetzung bei einer Temperatur von 55 bis 110°C, vorzugsweise 70 bis 110°C, insbesondere 80 bis 100°C durchgeführt.

Das Amin II und der Ester III werden in etwa äquimolarem Verhältnis zueinander eingesetzt, wobei ein leichter Überschuß einer der beiden Komponenten, etwa bis zu 5 mol-% noch tolerabel ist.

Die übliche Dauer für die Zudosierung des Amins II zum Ester III beträgt 15 min bis 4 Stunden, insbesondere 30 min bis 3 Stunden. Nach beendeter Zugabe des Amins II wird oftmals noch 30 min bis 3 Stunden bei der angegebenen Temperatur nachgerührt, bei rascher Abreaktion der Reaktanten kann aber auch auf ein Nachrühren verzichtet werden.

Das erfindungsgemäße Verfahren wird gemäß Maßnahme (c) in Abwesenheit von organischen, insbesondere von polaren organischen Lösungsmitteln durchgeführt. Bei der Reaktion entstehender Alkohol und eventuell in kleinen Mengen durch Hilfsstoffe eingebrachte Lösungsmittel werden sofort durch Destillation wieder entfernt.

Da das erfindungsgemäße Verfahren durch langsame Zugabe einer Schmelze des Amins II zum flüssigen Ester III unter rascher Abreaktion durchgeführt wird, liegt zu jedem Zeitpunkt ein homogenes einphasiges Reaktionsgemisch vor, was die Reaktionsführung besonders einfach gestaltet.

Der Polyhydroxyalkylrest Z leitet sich von Monosacchariden wie Erythrose, Threose, Ribose, Arabinose, Xylose, Lyxose, Allose, Altrose, Glucose, Mannose, Gulose, Idose, Galactose, Talose oder Fructose oder Derivaten hiervon wie Glucuronsäure oder Desoxyribose oder von Oligosacchariden, insbesondere von Disacchariden wie Saccharose, Lactose, Trehalose, Maltose, Cellobiose oder Gentiobiose, daneben auch von Trisacchariden wie Raffinose, ab. Weiterhin kommen alle technischen Stärkeabbauprodukte wie Glucosesirup oder Dextrine, z.B. Maltodextrine, in Betracht.

Bevorzugt wird für die Variable Z ein von Aldohexosen abgeleiteter Polyhydroxyalkylrest der Formel -CH₂-(CHOH)₄-CH₂OH. Besonders bevorzugt wird der Rest der Glucose, insbesondere der natürlich vorkommenden D-Glucose.

Die als Ausgangsmaterialien eingesetzten Amine II lassen sich bekanntermaßen in einfacher Weise durch reduktive Aminierung der genannten Zucker oder Zuckerderivate mit Ammoniak oder Alkylaminen R¹-NH₂ herstellen.

Der Rest R¹ bedeutet Wasserstoff oder C₁- bis C₈-Alkyl wie Methyl, Ethyl, n-Propyl, iso-Propyl, n-Butyl, iso-Butyl, sec.-Butyl, tert.-Butyl, n-Pentyl, n-Hexyl, n-Heptyl, n-Octyl oder 2-Ethylhexyl. Bevorzugt wird hiervon C₁-bis C₄-Alkyl, insbesondere Methyl, Ethyl, n-Propyl und n-Butyl.

Der Rest R² bezeichnet C₁- bis C₂₁-Alkyl, vorzugsweise C₂- bis C₂₁-Alkyl, vor allem C₅-bis C₁₉-Alkyl, insbesondere C₇-bis C₁₇-Alkyl. R² steht vorzugsweise für den Rest einer langkettigen Carbonsäure, insbesondere einer natürlich vorkommenden Fettsäure wie Laurinsäure, Myristinsäure, Palmitinsäure oder Stearinsäure oder einer synthetisch nach der Oxo- oder der Ziegler-Methode hergestellten Carbonsäure. Es können auch Gemische verschiedener Reste R² auftreten.

Der Rest R³ bedeutet C₁- bis C₄-Alkyl wie Methyl, Ethyl, n-Propyl, iso-Propyl, n-Butyl, iso-Butyl, sec.-Butyl oder tert.-Butyl, bevorzugt werden hiervon Methyl und Ethyl.

Als basische Katalysatoren eignen sich vor allem Alkalimetallhydroxide wie Natrium- oder Kaliumhydroxid, Alkalimetallcarbonate wie Natrium- oder Kaliumcarbonat und Alkalimethallalkoholate wie Natrium- oder Kaliummethanolat, Natrium- oder Kaliumethanolat oder Natrium- oder Kaliumisopropylat. Die Katalysatoren werden in den hierfür üblichen Mengen zugesetzt, also etwa 1 bis 25 mol-%, bezogen auf die Ausgangsverbindungen. Die genannten Katalysatoren können in fester Form oder im Falle der Alkoholate als etwa 10 bis 40 gew.-%ige, insbesondere 20 bis 30 gew.-%ige Lösungen in den entsprechenden Alkoholen zugegeben werden. Die Zugabe des Katalysators erfolgt entweder auf einmal zum vorgelegten Ester III vor dem Zudosieren des Amins II oder vorzugsweise portionsweise oder kontinuierlich während des Zudosierens des Amins II zum Ester III.

Die Umsetzung läuft in der Regel reibungsloser und vollständiger ab, wenn zusätzlich ein übliches Dispergiermittel in den hierfür üblichen Mengen mitverwendet wird. Hierzu eignen sich beispielsweise Propylenglykol oder das Produkt der Umsetzung selbst, also ein N-Alkanoyl-polyhydroxyalkylamin I. Insbesondere kommen hierzu jedoch Substanzen, die selbst oberflächenaktive Eigenschaften zeigen, in Betracht. So kann man nach dem erfindungsgemäßen Verfahren Gemische aus den Verbindungen I und weiteren oberflächenaktiven Verbindungen (Tensiden) herstellen und diese Gemische direkt einer Verwendung, beispielsweise auf dem Wasch- und Reinigungsmittelsektor, zuführen.

Als derartige oberflächenaktive Dispergiermittel eignen sich vor allem Alkoxylate von C₈- bis C₂₀-Alkoholen, insbesondere Ethoxylate und Propoxylate von üblichen Fettalkoholen oder Oxoalkoholen mit etwa 2 bis etwa 15 Einheiten Alkylenoxid.

Die beschriebenen Dispergiermittel werden üblicherweise in einer Menge von 10 bis 300 Gew.-%, vorzugsweise 30 bis 200 Gew.-%, bezogen auf eingesetzten Ester III, mitverwendet.

Nach beendeter Umsetzung wird der eingesetzte Katalysator zweckmäßigerweise neutralisiert, beispielsweise mit einer schwachen organischen Säure wie Citronensäure.

Das erfindungsgemäße Verfahren kann sowohl diskontinuierlich als auch kontinuierlich, beispielsweise in einer Rührkesselkaskade, durchgeführt werden.

Mit dem erfindungsgemäßen Verfahren erhält man farblose bis hell gefärbte Produkte mit einem hohen Gehalt an I und einem deutlich geringerem Spektrum an Ausgangsmaterialien und Nebenprodukten als bei den üblichen Verfahren des Standes der Technik. Als Nebenprodukte treten bei diesen meist cyclische N-Alkanoyl-polyhydroxyalkylamine, entstanden durch intramolekularen Ringschluß im Zuckerteil des Moleküls I, und Esteramide, entstanden durch Reaktion von I mit einem weiteren Molekül III unter Veresterung an einer Hydroxylgruppe im Zuckerteil von I, auf.

Die nach dem erfindungsgemäßen Verfahren erhaltenen Produkte bedürfen für die meisten Anwendungszwecke keiner weiteren Reinigung. Die hohe Reinheit und die selektive Bildung der N-Alkanoylpolyhydroxyalkylamine I ist hauptsächlich darauf zurückzuführen, daß die Umsetzung bei Temperaturen deutlich unter dem Schmelzbereich der Amine II durchgeführt wird und das Reaktionsprodukt so einer wesentlich geringeren thermischen Belastung ausgesetzt ist als bei den meisten bekannten Verfahren.

Besonders vorteilhaft ist beim erfindungsgemäßen Verfahren, daß man während des gesamten Reaktionsverlaufes in homogener, niedrigviskoser Phase arbeitet, wodurch stets eine gute Durchmischung gewährleistet ist, die Reaktionsführung und -kontrolle sich besonders einfach gestaltet und es nicht zu Verklumpungen und Anbackungen im Reaktor kommt. Dadurch ist das erfindungsgemäße Verfahren auch für eine großtechnische Produktion von N-Alkanoyl-polyhydroxyalkylaminen I geeignet.

Ein weiterer Vorteil des erfindungsgemäßen Verfahrens ist der Verzicht auf organische Lösungsmittel, die einen Verfahrens- und damit Energiemehraufwand und häufig auch Umweltprobleme mit sich bringen.

### Beispiele 1 bis 4 und Vergleichsbeispiel A

### Herstellung von N-Lauroyl-N-methylglucamin

### Beispiel 1

In einem Reaktor wurden 327 g (1,5 mol) Laurinsäuremethylester und 27 g einer 30 gew.%igen methanolischen Natriummethanolat-Lösung (0,15 mol NaOCH₃) vorgelegt und auf 80°C erwärmt. Unter reduziertem Druck (80 mbar) wurden eine 130°C heiße Schmelze von 293 g (1,5 mol) N-Methylglucamin und 54 g Propylenglykol innerhalb von 40 min zugetropft, so daß die Temperatur im Reaktor konstant bei 80°C blieb. Gleichzeitig wurde das bei der Reaktion gebildete Methanol zusammen mit dem zugegebenen Methanol aus der Katalysatorlösung im Vakuum fortlaufend abdestilliert. Die Reaktionsmischung wurde 1 bis 2 h bei 80°C nachgerührt und anschließend mit 14,1 g Citronensäure neutralisiert. Man erhielt ein helles, wachsartiges Produkt mit einem Schmelzbereich von 70 bis 80°C.

### Beispiel 2

327 g (1,5 mol) Laurinsäuremethylester wurden zusammen mit 54 g Propylenglykol im Reaktor vorgelegt und auf 80°C erwärmt. Unter reduziertem Druck (80 mbar) wurden gleichzeitig 293 g (1,5 mol) N-Methylglucamin-Schmelze von 130°C und 27 g 30 gew.-%ige methanolische Natriummethanolat-Lösung (0,15 mol NaOCH₃) innerhalb von 45 min unter Konstanthalten der Temperatur bei 80°C zugetropft. Das bei der Reaktion freigesetzte sowie das mit dem Katalysator zugegebene Methanol wurden im Vakuum fortlaufend abdestillert. Nach beendeter Reaktion wurde noch 1 h bei 80°C nachgerührt und das Produkt anschließend mit 9,4 g Citronensäure neutralisiert. Man erhielt ein helles, wachsartiges Produkt mit einem Schmelzbereich von 70 bis 80°C.

### Beispiel 3

219 g (1 mol) Laurinsäuremethylester wurden zusammen mit 22 g nicht neutralisiertem N-Lauroyl-N-methylglucamin aus Beispiel 1 vorgelegt und auf 100°C erwärmt. Unter reduziertem Druck (80 mbar) wurden 195 (1 mol) g N-Methylglucamin-Schmelze von 130 °C und 18 g 30 gew.-%ige methanolische Natriummethanolat-Lösung (0,1 mol NaOCH₃) innerhalb von 35 min unter Konstanthalten der Temperatur bei 100°C zudosiert. Es wurde wie in Beispiel 2 beschrieben weitergearbeitet. Man erhielt ein gelblich gefärbtes, wachsartiges Produkt.

### Beispiel 4

220 g (1 mol) Laurinsäuremethylester wurden im Reaktor vorgelegt und auf 100°C erwärmt. Unter reduziertem Druck (80 mbar) wurden gleichzeitig 195 g (1 mol) geschmolzenes N-Methylglucamin von 130°C und 18 g 30 gew.-%ige methanolische Natriummethylat-Lösung (0,1 mol NaOCH₃) innerhalb von 45 min unter Konstanthalten der Temperatur bei 100°C zugetropft. Das bei der Reaktion gebildete Methanol wurde zusammen mit dem Methanol aus der Katalysatorlösung im Vakuum fortlaufend abdestilliert. Gegen Ende der Zugabe stieg die Viskosität des Reaktionsgemisches stark an. Nachdem das Methanol vollständig aus dem Reaktionsgemisch entfernt war, wurde das Produkt mit 9,4 g Citronensäure neutralisiert. Man erhielt ein braun gefärbtes wachsartiges Produkt.

### Vergleichsbeispiel A

98 g (0,5 mol) festes N-Methylglucamin und 110 g (0,5 mol) Laurinsäuremethylester wurden im Reaktor vorgelegt und bei 125°C geschmolzen. Es resultierten 2 Phasen. Anschließend wurde auf 170°C erhitzt und 1,5 h bei dieser Temperatur gerührt. Das bei der Reaktion gebildete Methanol wurde dabei abdestilliert. Man erhielt eine braun gefärbte klare Schmelze.

Aus der nachfolgenden Tabelle ist die Zusammensetzung der ertaltenen Produkte zu ersehen. Die Bestimmungen wurden gaschromatographisch vorgenommen, die Angaben sind Flächenprozente.

**Tabelle**

| Zusammensetzung der Produkte | | | | | |
|---|---|---|---|---|---|
| Beispiel | 1 | 2 | 3 | 4 | A |
| N-Methylglucamin | 0,31 | 0,60 | 1,71 | 4,08 | 7,98 |
| Laurinsäure/Laurinester | 3,28 | 2,27 | 2,01 | 2,47 | 7,73 |
| Lauroyl-glucamin linear | 93,04 | 95,60 | 92,18 | 91,06 | 60,72 |
| Lauroyl-glucamin cyclisch | 0,22 | 0,10 | 0,86 | 0,93 | 10,93 |
| Esteramide und sonstige Nebenprodukte | 3,15 | 1,43 | 3,24 | 1,46 | 12,74 |

## Patentansprüche

1. Verfahren zur Herstellung von N-Alkanoyl-polyhydroxyalkylaminen der allgemeinen Formel I 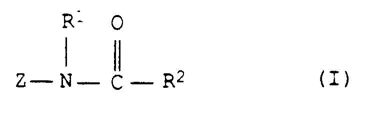 in der
Z für den Polyhydroxyalkylrest eines Mono- oder Oligosaccharides steht,
R¹ Wasserstoff oder C₁-C₈-Alkyl bedeutet und
R² C₁-bis C₂₁-Alkyl bezeichnet,
durch Umsetzung von Polyhydroxyalkylaminen der allgemeinen Formel II
Z-NH-R¹ (II)
mit Carbonsäurealkylestern der allgemeinen Formel III 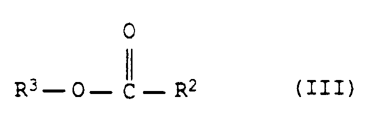 in der R³ einen C₁-bis C₄-Alkylrest bedeutet,
in Gegenwart eines basischen Katalysators, dadurch gekennzeichnet, daß man
(a) die Gesamtmenge des Esters III vorlegt, auf die Umsetzungstemperatur erwärmt und das Amin II in Form einer Schmelze unter Fortschreiten der Umsetzung zudosiert, wobei gebildeter Alkohol R³-OH fortlaufend abdestilliert wird,
(b) die Umsetzung bei einer Temperatur von 55 bis 110°C durchführt und
(c) die Umsetzung in Abwesenheit von organischen Lösungsmitteln vornimmt, ausgenommen wenn diese organischen Lösungsmittel in der Funktion als Dispergiermittel verwendet werden.

2. Verfahren nach Anspruch 1, dadurch gekennzeichnet, daß die Variable Z für einen Polyhydroxyalkylrest der Formel -CH₂-(CHOH)₄-CH₂OH steht.

3. Verfahren nach Anspruch 1 oder 2, dadurch gekennzeichnet, daß der Rest R¹ C₁-bis C₄-Alkyl bedeutet.

4. Verfahren nach den Ansprüchen 1 bis 3, dadurch gekennzeichnet, daß der Rest R² C₅-bis C₁₉-Alkyl bezeichnet.

5. Verfahren nach den Ansprüchen 1 bis 4, dadurch gekennzeichnet, daß man die Umsetzung bei einer Temperatur von 80 bis 100°C durchführt.

## Claims

1. A process for preparing N-alkanoylpolyhydroxyalkylamines of the general formula I 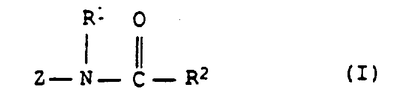 in which
Z represents the polyhydroxyalkyl radical of a mono- or oligosaccharide,
R¹ denotes hydrogen or C₁-C₈-alkyl and
R² signifies C₁- to C₂₁-alkyl,
by reacting polyhydroxyalkylamines of the general formula II
Z-NH-R¹ (II)
with alkyl carboxylates of the general formula III 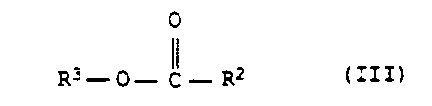 in which
R³ denotes a C₁- to C₄-radical,
in the presence of a basic catalyst, characterized in that
(a) the total amount of ester III is initially charged and heated to reaction temperature, and amine II is added metered in the form of a melt while the reaction is progressing and while the resulting alcohol R³-OH is continuously distilled off,
(b) the reaction is carried out at a temperature of from 55 to 110°C and
(c) the reaction is carried out in the absence of organic solvents, except when these organic solvents are used as dispersants.

2. A process according to Claim 1, characterized in that the variable Z represents a polyhydroxyalkyl radical of the formula -CH₂-(CHOH)₄-CH₂OH.

3. A process according to Claim 1 or 2, characterized in that the radical R¹ denotes C₁- to C₄-alkyl.

4. A process according to Claims 1 to 3, characterized in that the radical R² signifies C₅- to C₁₉-alkyl.

5. A process according to Claims 1 to 4, characterized in that the reaction is carried out at a temperature of from 80 to 100°C.

## Revendications

1. Procédé de préparation de N-alcanoyl-polyhydroxyalkylamines de formule générale I 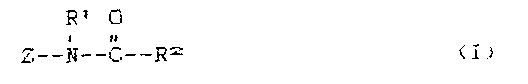 dans laquelle
Z est mis pour le reste polyhydroxyalkyle d'un mono- ou oligosaccharide,
R¹ représente un atome d'hydrogène ou un reste alkyle en C₁-C₈ et
R² représente un reste alkyle en C₁-C₂₁,
par réaction de polyhydroxyalkylamines de formule générale II
Z--NH--R¹ (II)
avec des esters alkyliques d'acide carboxylique de formule générale III 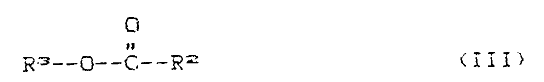 dans laquelle R³ représente un reste alkyle en C₁-C₄, en présence d'un catalyseur basique.
caractérisé en ce que
a) on introduit au préalable la quantité totale de l'ester III, on chauffe à la température de réaction et on ajoute l'amine II sous forme d'une masse fondue en maintenant le développement de la réaction, l'alcool R³-OH formé étant chassé de façon continue par distillation
b) on conduit la réaction à une température de 55 à 110°C et
c) on procède à la réaction en l'absence de solvants organiques, sauf si ces solvants organiques sont utilisés avec la fonction de dispersants.

2. Procédé selon la revendication 1, caractérisé en ce que la variable Z est mise pour un reste polyhydroxyalkyle de formule -CH₂-(CHOH)₄-CH₂OH,

3. Procédé selon la revendication 1 ou 2, caractérisé en ce que le reste R¹ représente un radical alkyle en C₁-C₄.

4. Procédé selon l'une quelconque des revendications 1 à 3, caractérisé en ce que le reste R² représente un radical alkyle en C₅-C₁₉.

5. Procédé selon l'une quelconque des revendications 1 à 4, caractérisé en ce que l'on conduit la réaction à une température de 80 à 100°C.
